# FASCICULE DE BREVET EUROPEEN

(11) **EP 1 240 212 B1**
(45) Date de publication et mention de la délivrance du brevet: **20.04.2005**
(21) Numéro de dépôt: 00966257.8
(22) Date de dépôt: 03.10.2000
(51) Int. Cl.: C08F 8/32, C08B 37/00, C08F 251/00, C08K 5/17, A61K 9/20

(54) **COPOLYMERES RETICULES A BASE DE COPOLYMERES POLYCARBOXYLIQUES NON RETICULES**
VERNETZTE COPOLYMERE AUF DER BASIS VON NICHT VERNETZTEN POLYCARBONSÄUREPOLYMEREN
CROSSLINKED COPOLYMERS BASED ON NON-CROSSLINKED POLYCARBOXYLIC COPOLYMERS

(30) Priorité: 04.10.1999 FR 9912363
(43) Date de publication de la demande: 18.09.2002
(73) Titulaire: SOCIETE DE CONSEILS DE RECHERCHES ET D'APPLICATIONS SCIENTIFIQUES (S.C.R.A.S.), 75016 Paris (FR)
(72) Inventeur: LABARRE, Denis, F-91140 Villebon-sur-Yvette (FR); LAMBERT, Nada, F-75016 Paris (FR); DUCOS, Cathy, F-28260 Anet (FR); DIANCOURT, Francis, F-28230 Epernon (FR)
(74) Mandataire: Bourgouin, André
(86) Numéro de dépôt international: PCT/FR2000/002731
(87) Numéro de publication internationale: WO 2001/025295

(56) Documents cités:
- WO-A-00/27886
- WO-A-86/01214
- WO-A-89/02445
- WO-A-91/16881
- WO-A-98/08897
- US-A- 5 017 229
- US-A- 5 219 971

## Description

L'invention concerne des copolymères réticulés à base de copolymères polycarboxyliques non réticulés, les dits copolymères non réticulés contenant au moins un polysaccharide. L'invention concerne également un procédé de préparation de ces copolymères et leur utilisation notamment comme support dans les compositions pharmaceutiques.

L'invention a ainsi pour objet des copolymères réticulés à base de copolymères polycarboxyliques non réticulés et d'un agent de réticulation comprenant aux moins deux fonctions aminés ; chaque copolymère polycarboxylique non réticulé comprend au moins un polysaccharide non réticulé lié par liaison covalente à au moins un autre polymère non réticulé non saccharidique. Enfin au moins un des polysaccharides et polymères non saccharidiques, constituants du même copolymère non réticulé, est polycarboxylique.

Dans la demande WO98/08897, la déposante a revendiqué des copolymères réticulés, à base de polymères polycarboxyliques non réticulés, les dits copolymères contenant au moins un polysaccharide polycarboxylique. Ainsi, un coplymère selon cette demande internationale précitée, contient au moins un polysaccharide polycarboxylique et au moins un autre polymère polycarboxylique qui n'est pas un polysaccharide (lignes 16-18 de la page 1 de la demande W098/08897). Cependant, le procédé qui consiste à mélanger en solution aqueuse les polymères polycarboxyliques des deux types (polysaccharidiques et non polysaccharidiques), ne permet pas d'exclure l'existence, dans le copolymère réticulé final, d'hétérogénéités résultant de réactions de réticulation soit entre polysaccharides uniquement, soit entre polymères carboxyliques non polysaccharidiques uniquement.

La présente demande propose donc de résoudre ce problème en préparant tout d'abord des copolymères entre les deux espèces de départ (polymère polysaccharide polycarboxylique d'une part et polymère polycarboxylique non-saccharidique d'autre part), puis à réticuler les copolymères ainsi obtenus ; ceci permet d'exclure d'éventuelles hétérogénéités dans la mesure où une liaison covalente pré-existe à la réaction de réticulation. L'invention propose donc de nouveaux copolymères réticulés à base de copolymères polycarboxyliques non réticulés.

L'association d'un polysaccharide avec un autre type de polymère, permet de moduler les propriétés des polysaccharides telles que l'hydrophilie. On peut ainsi obtenir des copolymères avec des propriétés de dégradation appropriées en fonction de leurs applications. Par ailleurs, les copolymères selon l'invention, sont avantageusement préparés en milieu aqueux.

Selon l'invention, le polysaccharide peut être polycarboxylique ou non. De même, le polymère non saccharique peut être polycarboxylique ou non. Si l'un de ces deux polymères est non polycarboxylique, l'autre est nécessairement polycarboxylique afin de rendre possible la réticulation. Les deux polymères saccharidique et non saccharidique peuvent être tous les deux polycarboxyliques.

Les polysaccharides non polycarboxyliques non réticulés peuvent être choisis, par exemple, parmi l'agarose, l'agaropectine, l'amylose, l'amylopectine, l'arabino-galactane, les carraghénanes, la cellulose ou la méthylcellulose, le chitosane, le dextrane, le sulfate de kératane, les fucanes et fucoïdanes, les gommes d'adragante, arabiques, de caroube et de guar, ou le pullulane, ou encore leurs dérivés substitués non carboxyliques.

Les polysaccharides polycarboxyliques non réticulés peuvent être choisis, par exemple, parmi les glycosaminoglycanes, l'acide pectinique (pectine), l'acide alginique (alginate), les poly(acides sialiques) tels que l'acide colominique, le xanthane, les dérivés carboxyliques des polysaccharides non carboxyliques cités précédemment et notamment ceux du dextrane tels que les carboxyméthyldextranes et leurs dérivés, ou encore les dérivés carboxyliques de la cellulose tels que les carboxyméthylcelluloses. Parmi les glycosaminoglycanes, on peut citer l'acide hyaluronique et ses dérivés, la chondroïtine sulfate, l'héparine, le sulfate de dermatane, le sulfate d'héparane ou un mélange de ces derniers.

Parmi les polymères polycarboxyliques non réticulés non saccharidiques, on peut citer le poly(acide glutamique), le poly(acide aspartique), le poly(acide maléique), le poly(acide succinique), le poly(acide itaconique), le poly(acide malique) ou le poly(acide fumarique), les polymères acryliques polycarboxyliques tels que le poly(acide acrylique), le poly(acide méthacrylique) ou les copolymères de ces derniers tels que les Eudragits® L et S.

Parmi les polymères non polycarboxyliques non réticulés et non saccharidiques, on peut citer le poly(acétate de vinyle), le poly(alcool vinylique), les poly(esters acryliques), les poly(esters méthacryliques), les poly(méthacrylamides) et les poly(acrylamides).

L'expression polymères polycarboxyliques, saccharidiques ou non, comprend les polymères tels que définis ci-dessus mais également les dérivés partiellement substitués de ces polymères comme, par exemple, les esters, les amides et les dérivés partiellement ou totalement substitués de ces polymères comme les sels de ces polymères polycarboxyliques.

L'invention a plus particulièrement pour objet des copolymères réticulés tels que définis ci-dessus, caractérisés en ce que le polysaccharide est polycarboxylique. De préférence, il est choisi parmi l'acide pectinique (pectine), l'acide alginique (alginate), les glycosaminoglycanes, et de préférence l'acide hyaluronique, la chondroïtine sulfate, l'héparine, le sulfate de dermatane, le sulfate d'héparane ou un mélange de ces derniers.

L'invention a plus particulièrement pour objet des copolymères réticulés tels que définis ci-dessus, caractérisés en ce que le polysaccharide est non polycarboxylique. De préférence, il est choisi parmi l'agarose, l'agaropectine, l'amylose, l'amylopectine, l'arabino-galactane, les carraghénanes, la cellulose ou la méthylcellulose, le chitosane, le dextrane, le sulfate de kératane, les fucanes et fucoïdanes, les gommes d'adragante, arabiques, de caroube et de guar, ou le pullulane.

L'invention a plus particulièrement pour objet des copolymères réticulés tels que définis ci-dessus, caractérisés en ce que le polymère non réticulé non saccharidique est polycarboxylique. De préférence, il choisi parmi les polymères acryliques polycarboxyliques, et plus particulièrement le poly(acide acrylique) ou le poly(acide méthacrylique).

L'invention a plus particulièrement pour objet des copolymères réticulés tels que définis ci-dessus, caractérisés en ce que le polymère non réticulé non saccharidique est non polycarboxylique. De préférence, il est choisi parmi le poly(acétate de vinyle), le poly(alcool vinylique), les poly(esters acryliques), les poly(esters méthacryliques), les poly(méthacrylamides) et les poly(acrylamides).

Les copolymères polycarboxyliques non réticulés selon l'invention, sont liés entre eux par un agent de réticulation. Cet agent de réticulation comprend au moins deux fonctions amines qui sont susceptibles de réagir avec les fonctions carboxyliques libres des dits copolymères carboxyliques non réticulés. Il peut être choisi, par exemple, parmi les protéines, les polyamines, les triamines, les diamines, les acides aminés naturels ou synthétiques, ou les dérivés des composés tels que définis ci-dessus comme, par exemple, leurs sels, leurs esters ou leurs amides. Parmi les acides aminés, on peut citer, par exemple, l'arginine, la lysine, l'histidine et l'ornithine. Parmi les diamines, on peut citer l'éthylènediamine, la butanediamine, l'hexanediamine, l'heptanediamine, l'octanediamine ou la dodécanediamine. Parmi les polyamines, on peut citer le chitosanc, les poly(éthylène imines), les poly(acide aminé) tels que la polylysine ou la polyomithine, ainsi que les copolymères de ces polyamines. L'agent de réticulation peut également être choisi parmi les composés tels que la spermine, la spermidine, la mélamine, la guanidine ou la diéthylènetriamine. L'invention a également pour objet des copolymères tels que définis ci-dessus, dans lesquels l'agent de réticulation est choisi parmi les diamines, les acides aminés naturels ou synthétiques ou les polyamines, et préférentiellement les diamines. De préférence, l'agent de réticulation utilisé est une diamine et avantageusement l'hexanediamine.

L'invention a plus particulièrement pour objet également des copolymères réticulés tels que définis ci-dessus, caractérisés en ce que le polysaccharide est un polysaccharide dégradable par la flore microbienne du colon tel que la chondroïtine sulfate, l'acide hyaluronique, l'acide pectinique, l'héparine, le dextrane, le chitosane, l'amylose, la pectine, les alginates ou le xanthane, et plus particulièrement la chondroïtine sulfate ou le chitosane.

L'invention a plus particulièrement pour objet des copolymères réticulés tels que définis ci-dessus, caractérisés en ce que le polysaccharide est la chondroïtine sulfate, le polymère non saccharidique est choisi parmi le poly(acide acrylique) et le poly(acide méthacrylique), et l'agent de réticulation est l'hexanediamine.

L'invention a également pour objet un procédé de préparation de copolymères réticulés tels que définis ci-dessus, ledit procédé caractérisé en ce que l'on fait réagir les dits copolymères polycarboxyliques non réticulés constituant le copolymère réticulé, en présence d'un activateur et d'un agent de réticulation comprenant au moins deux fonctions amine, dans un milieu réactionnel approprié. De préférence, la préparation de copolymères réticulés tels que définis ci-dessus s'effectue en milieu aqueux. L'expression milieu aqueux signifie un milieu ne contenant que de l'eau ou de l'eau mélangée avec un ou plusieurs solvants miscibles à l'eau tel que, par exemple, l'acétone, les alcools inférieurs tels que l'éthanol, ou les acides. D'autres agents tels que la N-hydroxysuccinimide, susceptibles de favoriser la réticulation, peuvent également être utilisés. De préférence le milieu aqueux ne comprend que de l'eau. La mise en oeuvre du procédé selon l'invention peut s'effectuer de différentes manières en faisant varier l'ordre de mise en réaction des différents réactifs. En effet, le procédé peut consister à mélanger ensemble les copolymères polycarboxyliques non réticulés et l'agent de réticulation, puis à rajouter l'activateur. Le procédé de réticulation selon l'invention peut consister également à mélanger ensemble les copolymères polycarboxyliques non réticulés et l'activateur, puis à rajouter l'agent de réticulation. Le procédé peut consister également à réticuler un des copolymères polycarboxyliques non réticulés constituant du copolymère réticulé, en mélangeant ce dit copolymère non réticulé avec l'agent de réticulation puis l'activateur ou bien avec l'activateur puis l'agent de réticulation, puis à rajouter dans le milieu réactionnel au moins un autre copolymère polycarboxylique non réticulé, pour le réticuler avec le dit copolymère présent dans le mélange réactionnel. Lors de la mise en oeuvre du procédé, les réactifs mis en présence peuvent être préalablement solubilisés dans le milieu réactionnel choisi. De préférence, les copolymères polycarboxyliques non réticulés et l'agent de réticulation sont mélangés ensemble dans un milieu aqueux jusqu'à solubilisation puis l'activateur est rajouté. Le procédé est mis en oeuvre à une température comprise entre -30 et 100° C, de préférence entre 0 et 40° C et de manière très préférentielle à 4° C. La température de mise en oeuvre du procédé de réticulation est bien évidemment inférieure aux températures de dégradation ou de décomposition des réactifs mis en présence.

Les proportions relatives des réactifs que sont les copolymères polycarboxyliques non réticulés, l'agent de réticulation et l'activateur, peuvent varier selon les caractéristiques des copolymères recherchés. Les copolymères polycarboxyliques non réticulés peuvent varier dans un rapport molaire compris entre 0,01 et 100. Le rapport molaire de l'agent de réticulation par rapport aux fonctions carboxyliques totales peut varier entre 0,01 et 100. Le rapport molaire de l'activateur par rapport aux fonctions carboxyliques totales peut varier entre 0,01 et 100.

L'activateur peut être choisi parmi les agents de couplage classiquement utilisés en synthèse peptidique. Ainsi l'activateur peut être choisi, par exemple, parmi les carbodiimides, des dérivés des quinolines ou des anhydrides mixtes. Comme exemple de carbodiimides, on peut citer les hydrohalogénures tels que l'hydrochlorure de N-(3-diméthylaminopropyl)-N'-éthyl carbodiimide (EDC), le N-cyclohexyl-N'-(2-morpholinoéthyl) carbodiimide (CMC). Comme exemple des dérivés des quinolines, on peut citer la 2-éthoxy-N-éthoxycarbonyl-1,2-dihydroquinoline (EEDQ), la N-isobutoxycarbonyl-2-isobutoxy-1,2-dihydroquinoline (IIDQ), la N-isobutoxycarbonyl-2-méthoxy-1,2-dihydroquinoline (IMDQ), la N-isobutoxycarbonyl-2-éthoxy-1,2-dihydroquinoline (IEDQ). Comme exemple d'anhydrides mixtes, on peut citer les chloroformates et plus particulièrement l'isobutylchloroformate (IBC). De préférence, l'activateur utilisé est l'hydrochlorure de N-(3-diméthylaminopropyl)-N'-éthyl carbodiimide. L'invention a également pour objet un procédé de préparation de copolymères réticulés tels que définis ci-dessus, dans lequel l'activateur est choisi parmi les carbodiimides, les dérivés des quinolines et les anhydrides mixtes.

Les copolymères non réticulés peuvent être obtenus par exemple par couplage des polymères correspondants ou par polymérisation radicalaire à partir du polysaccharide et du monomère du polymère non saccharidique.

L'invention a également pour objet un procédé de préparation des copolymères non réticulés tels que définis ci-dessus, ledit procédé caractérisé en ce que l'on greffe sur le polysaccharide, en milieu aqueux sous atmosphère inerte et en présence d'un catalyseur, le monomère du polymère non saccharidique, monomère qui va ensuite polymériser dans ces conditions réactionnelles. L'expression milieu aqueux signifie un milieu ne contenant que de l'eau ou de l'eau mélangée avec un ou plusieurs solvants miscibles à l'eau tel que, par exemple, l'acétone, les alcools inférieurs tels que l'éthanol ou bien les acides. La valeur du pH du milieu réactionnel est adaptée aux réactifs mis en jeu. Le catalyseur utilisé est choisi parmi les catalyseurs communément utilisés par l'homme de l'art comme par exemple les ions cériques. Le procédé est mis en oeuvre à une température comprise entre -30 et 100° C, de préférence entre 20 et 60° C et de manière très préférentielle à 40° C. La température de mise en oeuvre du procédé est bien évidemment inférieure aux températures de dégradation ou de décomposition des réactifs mis en présence.

Les proportions relatives des réactifs que sont les polysaccharides, les monomères des polymères non saccharidiques et l'agent catalytique, peuvent varier selon les caractéristiques des copolymères réticulés recherchés. Les proportions des réactifs sont définies en fonction des masses moléculaires souhaitées des copolymères polycarboxyliques non réticulés. Les polymères non réticulés peuvent varier dans un rapport d'unité compris entre 0,01 et 100 par rapport aux unités de saccharide.

Les copolymères réticulés selon l'invention peuvent être utilisés, par exemple, dans les domaines pharmaceutiques, cosmétiques, biomédicaux, vétérinaires, chimiques, agrochimiques ou agroalimentaires.

Plus particulièrement, l'invention a pour objet une composition pharmaceutique contenant au moins un principe actif et, à titre de support inerte ou d'excipient, au moins un copolymère réticulé selon l'invention. L'expression principe actif désigne toute substance ou mélange de substances ayant une activité thérapeutique.

Une telle composition peut être élaborée à partir de ces différents composants par toute technique classique connue de l'homme de l'art. Elle peut se présenter, par exemple, sous forme de comprimés matriciels, de comprimés enrobés par les copolymères de la présente invention, de comprimés multicouches, de pellets matriciels, de pellets ou des microparticules enrobés par les copolymères de la présente invention. Ces microparticules et pellets peuvent être contenus ou non dans des capsules. Elle peut se présenter également sous forme de microparticules ou de nanoparticules dont l'un au moins des constituants est un copolymère de la présente invention ou bien sous toute autre forme permettant une administration orale. Elle peut se présenter également sous toute autre forme adaptée au mode d'administration choisi ou approprié telle que des suppositoires ou des préparations pour application locale ou pour injection. La quantité du principe actif permettant une action pharmacologique efficace, en particulier thérapeutique, peut varier en fonction de la nature du principe actif, de l'âge et/ou de la maladie du patient à traiter.

Grâce à son réseau réticulé, un copolymère selon l'invention peut être utilisé pour une libération prolongée du principe actif. La présente invention a donc également pour objet l'utilisation d'une composition pharmaceutique selon l'invention pour une libération contrôlée du ou des principes actifs qu'elle contient.

De telles compositions peuvent également posséder d'autres caractéristiques qui dépendent éventuellement des caractéristiques des polymères de départ telles que la bioadhésion. Ainsi, une composition pharmaceutique selon l'invention peut également être utilisée en tant que système pharmaceutique bioadhésif. La présente invention a donc également pour objet l'utilisation d'une composition pharmaceutique selon l'invention en tant que système bioadhésif.

Des compositions telles définies ci-dessus dans lesquelles le polysaccharide est dégradable par la flore du colon, peuvent également être utilisée en tant que système à libération spécifique au niveau du colon par action de la flore microbienne. Le concept de la libération spécifique au niveau du colon par action de la flore microbienne, est basé sur la propriété du colon de posséder une flore microbienne très abondante qui, de plus, a la potentialité de métaboliser des substances faiblement ou non dégradées par la partie haute du tube digestif. De telles compositions sont particulièrement adaptées pour véhiculer des principes actifs destinés au traitement des maladies du colon, ce qui permet d'augmenter leur efficacité et de diminuer leurs effets secondaires. Parmi ces principes actifs figurent les stéroïdes telles que la dexamethasone et l'hydrocortisone, les anti-inflammatoires non stéroïdiens tels que l'acide 5-aminosalicylique, les antinéoplasiques tels que le methotrexate, le tamoxifène, les antispasmodiques et les agents chimiothérapiques. De telles compositions sont particulièrement adaptées également pour véhiculer des principes actifs qui sont absorbés de façon plus efficace au niveau du colon tels que les stéroïdes ou la xanthine. Leur administration directe au niveau du colon permet d'augmenter leur efficacité. De telles compositions sont particulièrement adaptées également pour véhiculer des principes actifs qui sont dégradés dans les parties hautes du tube digestif. Parmi ces principes actifs, on peut citer les peptides et les protéines d'origine naturelle ou de synthèse ainsi que les fragments pharmacologiquement actifs tels que les vaccins oraux, l'insuline, les peptides contraceptifs, les peptides activateurs du plasminogène, les peptides de croissance et autres peptides participant aux régulations hormonales.

Les exemples suivants sont présentés pour illustrer les procédures ci-dessus et ne doivent en aucun cas être considérés comme une limite à la portée de l'invention.

### PARTIE EXPÉRIMENTALE

### Synthèse des copolymères non réticulés

### Exemple 1 : synthèse d'un copolymère chondroïtine sulfate -co- acide polyméthacrylique

Dans un erlen bouché, sont dissout à 40°C 250 mg de chondroïtine sulfate (CS) dans 50 ml d'une solution de HNO₃ 0,2 M préalablement dégazée. On y ajoute 3,625 ml d'une solution d'ions cérium (de concentration 8.10⁻² mole/litre dans HNO₃ 0,2 M préalablement dégazée) et 2,5 ml d'acide méthacrylique. La réaction s'effectue à 40° C pendant environ 50 minutes sous agitation. Après refroidissement, le pH est ramené à 7 avec de la soude. Une solution de citrate 1 M est ajoutée. Le milieu final est alors filtré sur une membrane de taille de pore 10 kilo-dalton par diafiltration tangentielle. L'analyse du copolymère purifié peut être effectuée sur la solution ainsi obtenue ou après une étape de lyophilisation.

La masse moléculaire du copolymère est évaluée par chromatographie par perméation de gel en phase aqueuse (milieu NaCl 0,2 N). Les colonnes sont étalonnées avec des standards de dextrane de masse moléculaires comprises entre 40 000 et 400 000.

Les masses sont exprimées en masse moléculaire moyenne en masse (Mw) et masse moléculaire au pic du chromatogramme, représentant la majorité de la population macromoléculaire (Mp). L'indice de polydispersité (Ip), correspondant au rapport Mw/Mn (masse moléculaire moyenne en nombre), représente la dispersion des masses sur l'ensemble du copolymère.

Copolymère 1 :
Mw = 170 000
Mp = 78 000
Ip = 5.38

Le nombre des groupements carboxyles présents est déterminé par dosage conductimétrique. La solution de copolymère à doser est préalablement éluée sur une résine échangeuse d'ions de type sulfonique (DOWEX 50Wx8). Cette étape préparative permet d'obtenir les copolymères sous forme acide et de doser distinctement toutes les acidités sulfates et carboxylates d'origine polysaccharidique ainsi que les acidités carboxylates d'origine polycarboxylique non saccharidique. La neutralisation de la solution par la soude 0,1 N est ensuite suivie par conductimétrie. Les résultats sont exprimés en méqNaOH (mg de NaOH/g de copolymères).

Copolymère 1 :
- 1,39.10⁺² meqNaOH (PMA+CS),
- 8,94.10⁺¹ meqNaOH (PMA) ;
- 5,08.10⁺¹ meqNaOH (CS).

Le spectre RMN-¹³C du copolymère (PMA+CS) comprend les pics caractèristiques de la CS et du PMA, à savoir respectivement le massif entre 51 et 105 ppm correspondant aux carbones portés par les glucuroniques et galactosamine, le pic caractéristique du N-acétyl centré à 175 ppm, et les signaux correspondant aux méthyles (19,2 ppm) et aux carboxyles (186,4 ppm) de l'acide polyméthacrylique. Mais ce spectre comprend également les signaux qui n'appartiennent à aucune des deux entités (119,8 et 181,4 ppm) et que l'on attribue aux liaisons entre la CS et le PMA. Cette analyse met en évidence la polymérisation de l'acide méthacrylique sur la CS.

### Exemple 2 : synthèse de copolymères chondroïtine sulfate -co- acide polyméthacrylique de masse moléculaires et ratio CS/PMA variables.

Des masses moléculaires ainsi que des ratio CS/PMA différents peuvent être obtenus en modifiant les proportions de réactifs introduits et notamment :
- modification de la quantité de cérium.
- modification de la quantité d'acide méthacrylique
- modification de la molarité de la solution d'acide nitrique.

Ces études montrent des différences de masses significatives entre les polymère synthétisés (données GPC).

Dans un erlen bouché, sont dissout à 40° C 250 mg de chondroïtine sulfate dans 50 ml d'une solution de HNO₃ *X* M préalablement dégazée. On y ajoute *Y* ml d'une solution d'ions cérium.(de concentration 8.10⁻² mole/litre dans HNO₃ *X* M préalablement dégazée) et *Z* ml d'acide méthacrylique. La réaction s'effectue à 40° C pendant environ 50 minutes sous agitation. Après refroidissement, le pH est ramené à 7 avec de la soude. Une solution de citrate 1M est ajoutée. Le milieu final est alors filtré sur une membrane de taille de pore 10 kilo-dalton par diafiltration tangentielle.

L'analyse du copolymère purifié peut être effectuée sur la solution ainsi obtenue ou après une étape de lyophilisation.

Les conditions opératoires ainsi que la masse moléculaire des copolymères purifiés (déterminée par chromatographie aqueuse par perméation de gel) sont reportées sur le tableau 1 ci-dessous.

### Réticulation des copolymères synthétisés

### Exemple 3

100 mg du copolymère obtenu dans l'exemple 1, du diaminohexane et du N-hydroxysuccinimide sont dissous dans 2 ml d'une solution aqueuse contenant de la triéthylamine de façon à avoir un pH final de 8,5-9,5. On ajoute de l'EDC. On maintient le pH à 8,5-9,5 pendant 24 h à 4° C. On lave les précipités obtenus avec des solutions de NaCl 5 M puis avec de l'eau bidistillée. Puis on lyophilise les précipités puis on détermine la masse.

Les quantités de diaminohexane, d'EDC et de N-hydroxysuccinimide étudiées sont les suivantes

| Préparation | A | B | C |
|---|---|---|---|
| EDC(mg) | 320 | 170 | 170 |
| diaminohexane(mg) | 160 | 160 | 80 |
| hydroxysuccinimide(mg) | 310 | 310 | 155 |
| Masse du précipité (mg) | 180 | 17 | 80 |

### Exemple 4

On dissout à 4° C 100 mg du copolymère obtenu dans l'exemple 1, 160 mg de diaminohexane et 310 mg de N-Hydroxysuccinimide dans 2 ml de tampon borate de pH9. On ajoute 320mg d'EDC. On laisse la réaction se faire à 4° C pendant 24 heures sous agitation. On lave les précipités obtenus avec des solutions de NaCl 5 M puis avec de l'eau bidistillée. Puis on lyophilise les précipités. La masse du précipité obtenu est de 100 mg.

### Exemple 5

On dissout à 4° C 100 mg du copolymère obtenu dans l'exemple 1, 160 mg de diaminohexane et 310 mg de N-hydroxysuccinimide dans 2 ml de tampon bicarbonate de pH9. On ajoute 320 mg d'EDC. On laisse la réaction se faire à 4° C pendant 24 heures sous agitation. On lave les précipités obtenus avec des solutions de NaCl 5 M puis avec de l'eau bidistillée. Puis on lyophilise les précipités. La masse du précipité obtenu est de 50 mg.

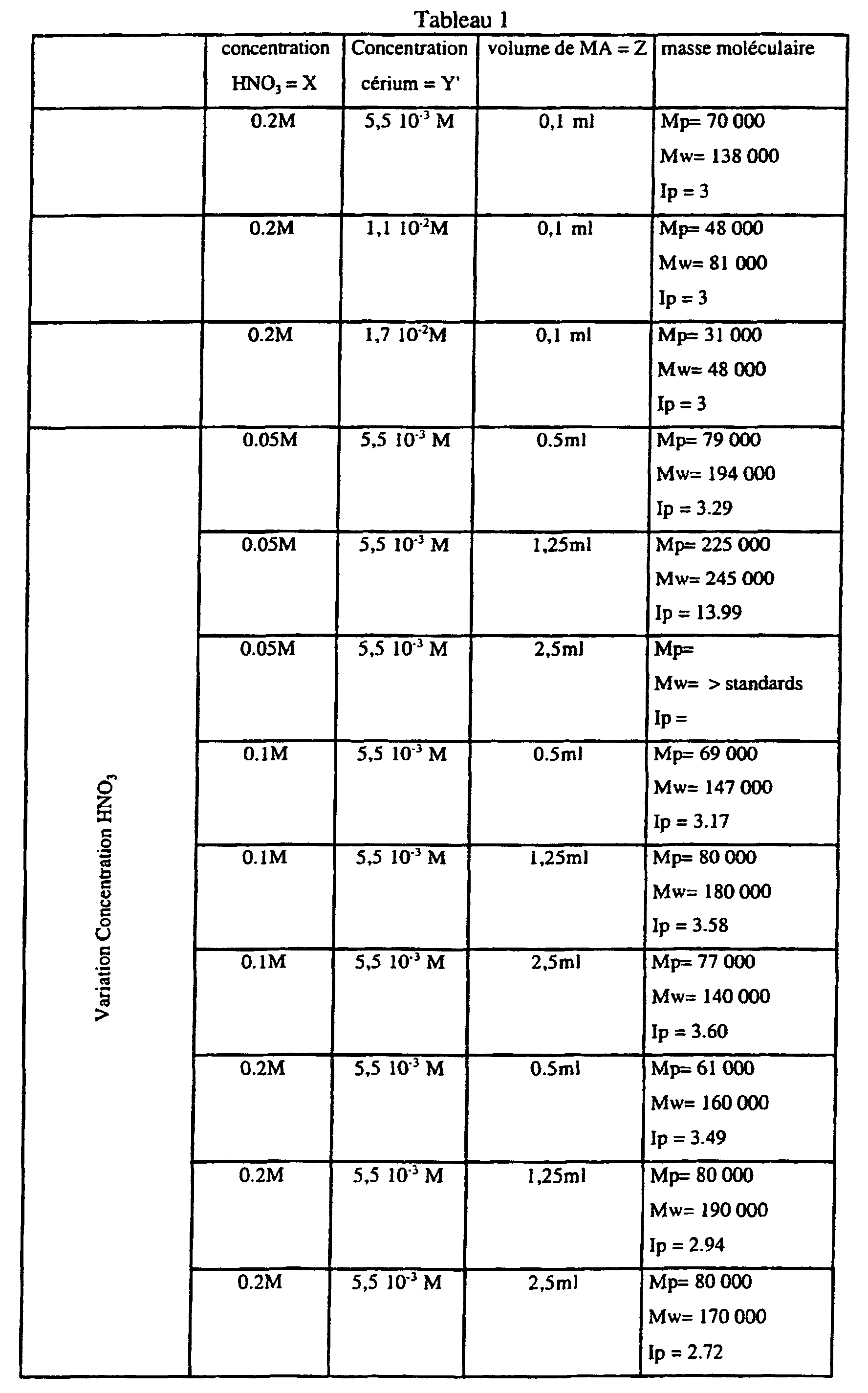

## Revendications

1. Copolymères réticulés à base de copolymères polycarboxyliques non réticulés et d'un agent de réticulation comprenant au moins deux fonctions amine,
chaque copolymère polycarboxylique non réticulé comprenant au moins un polysaccharide non réticulé lié par liaison covalente à au moins un autre polymère non réticulé non saccharidique, et
au moins un des polysaccharides et polymères non saccharidiques, constituants du même copolymère non réticulé, est polycarboxylique.

2. Copolymères selon la revendication 1, **caractérisés en ce que** le polysaccharide est non polycarboxylique.

3. Copolymères selon l'une des revendications 1 à 2, **caractérisés en ce que** le polysaccharide non polycarboxylique est choisi parmi l'agarose, l'agaropectine, l'amylose, l'amylopectine, l'arabino-galactane, les carraghénanes, la cellulose ou la méthylcellulose, le chitosane, le dextrane, le sulfate de kératane, les fucanes et fucoïdanes, les gommes d'adragante, arabiques, de caroube et de guar, ou le pullulane.

4. Copolymères selon la revendication 1, **caractérisés en ce que** le polysaccharide est polycarboxylique.

5. Copolymères selon l'une des revendications 1 ou 4, **caractérisés en ce que** le polysaccharide polycarboxylique est choisi parmi les glycosaminoglycanes, l'acide pectinique ou alginique.

6. Copolymères selon l'une des revendications 1, 4 ou 5, **caractérisés en ce que** le polysaccharide polycarboxylique est un glycosaminoglycane choisi parmi l'acide hyaluronique, la chondroïtine sulfate, l'héparine, le sulfate de dermatane et le sulfate d'héparane.

7. Copolymères selon la revendication 1 à 6, **caractérisés en ce que** le polymère non saccharidique est non polycarboxylique.

8. Copolymères selon l'une des revendications 1 à 7, **caractérisés en ce que** le polymère non saccharidique non polycarboxylique est choisi parmi, le poly(acétate de vinyle), le poly(alcool vinylique), les poly(esters acryliques), les poly(esters méthacryliques), les poly(méthacrylamides) et les poly(acrylamides).

9. Copolymères selon l'une quelconque des revendications 1 à 6, **caractérisés en ce que** le polymère non saccharidique est polycarboxylique.

10. Copolymères selon l'une quelconque des revendications 1 à 6 ou 9, **caractérisés en ce que** le polymère non saccharidique est un polymère acrylique polycarboxylique.

11. Copolymères selon la revendication 10, **caractérisés en ce que** le polymère acrylique polycarboxylique est le poly(acide acrylique) ou le poly(acide méthacrylique).

12. Copolymères selon l'une quelconque des revendications 1 à 11, dans lesquels l'agent de réticulation est choisi parmi les diamines, les acides aminés naturels ou synthétiques ou les polyamines, et préférentiellement les diamines.

13. Copolymères selon la revendication 12 dans lesquels l'agent de réticulation est une diamine.

14. Copolymères selon l'une des revendications 1 à 13, **caractérisés en ce que** le polysaccharide est dégradable par la flore microbienne du colon.

15. Copolymères selon la revendication 14, **caractérisés en ce que** le polysaccharide est choisi parmi la chondroïtine sulfate, l'acide hyaluronique, l'acide pectinique, l'héparine, le dextrane, le chitosane, l'amylose, la pectine, les alginates ou le xanthane.

16. Copolymères selon l'une quelconque des revendications 14 à 15, **caractérisés en ce que** le polysaccharide est la chondroïtine sulfate, l'autre dit polymère non saccharidique est le poly(acide acrylique) ou le poly(acide méthacrylique), et l'agent de réticulation est l'hexanediamine.

17. Procédé de préparation de copolymères réticulés selon l'une quelconque des revendications 1 à 16, **caractérisé en ce que** l'on fait réagir, en milieu aqueux, les dits copolymères polycarboxyliques non réticulés, en présence d'un activateur et dudit agent de réticulation.

18. Procédé selon la revendication 17, dans lequel l'activateur est choisi parmi les carbodiimides, les dérivés des quinolines et les anhydrides mixtes.

19. Procédé de préparation de copolymères non réticulés selon la revendication 1, **caractérisé en ce que** l'on greffe sur le polysaccharide, en milieu aqueux sous atmosphère inerte et en présence d'un catalyseur, le monomère du polymère non saccharique, monomère qui va ensuite polymériser dans ces conditions réactionnelles.

20. Composition pharmaceutique contenant au moins un principe actif et, à titre de support inerte ou d'excipient, au moins un copolymère réticulé selon l'une des revendications 1 à 13.

21. Composition pharmaceutique contenant au moins un principe actif et, à titre de support inerte ou d'excipient, au moins un copolymère selon l'une des revendications 14 à 16.

22. Utilisation d'une composition pharmaceutique selon l'une des revendications 20 à 21 pour une libération contrôlée.

23. Utilisation d'une composition pharmaceutique selon l'une des revendications 20 à 21 en tant que système pharmaceutique bioadhésif.

24. Utilisation d'une composition pharmaceutique selon la revendication 21 pour une libération spécifique du principe actif au niveau du colon.

25. Utilisation selon la revendication 24 pour véhiculer le principe actif destiné au traitement des maladies du colon.

26. Utilisation selon la revendication 24 pour véhiculer le principe actif qui est absorbé au niveau du colon.

27. Utilisation selon la revendication 24 pour véhiculer le principe actif qui est dégradé dans les parties hautes du tube digestif.

## Patentansprüche

1. Vernetzte Copolymere auf der Basis von nicht vernetzten polycarboxylischen Copolymeren und eines Vernetzungsmittels mit mindestens zwei Aminfunktionen,
wobei jedes nicht vernetzte polycarboxylische Copolymer mindestens ein nicht vernetztes Polysaccharid umfasst, das an mindestens ein anderes nicht vernetztes Nicht-Saccharid-Polymer durch kovalente Bindung gebunden ist, und
mindestens eines der Polysaccharide und Nicht-Saccharid-Polymere, die das nicht vernetzte Copolymer bilden, polycarboxylisch ist.

2. Copolymere nach Anspruch 1, **dadurch gekennzeichnet, dass** das Polysaccharid nicht-polycarboxylisch ist.

3. Copolymere nach einem der Ansprüche 1 bis 2, **dadurch gekennzeichnet, dass** das nicht-polycarboxylische Polysaccharid aus Agarose, Agaropectin, Amylose, Amylopectin, Arabino-galactan, Carrageenanen, Cellulose oder Methylcellulose, Chitosan, Dextran, Keratansulfat, Fucanen und Fucoidanen, Adragantgummi, Gummi Arabicum, Johannisbrotgummi, Guargummi oder Pullulan ausgewählt ist.

4. Copolymere nach Anspruch 1, **dadurch gekennzeichnet, dass** das Polysaccharid polycarboxylisch ist.

5. Copolymere nach einem der Ansprüche 1 oder 4, **dadurch gekennzeichnet, dass** das polycarboxylische Polysaccharid aus Glycosaminoglycanen, Pectinsäure oder Alginsäure ausgewählt ist.

6. Copolymere nach einem der Ansprüche 1, 4 oder 5, **dadurch gekennzeichnet, dass** das polycarboxylische Polysaccharid ein Glycosaminoglycan ist, das aus Hyaluronsäure, Chondroitinsulfat, Heparin, Dermatansulfat und Heparansulfat ausgewählt ist.

7. Copolymer nach Anspruch 1 bis 6, **dadurch gekennzeichnet, dass** das Nicht-Saccharid-Polymer nicht-polycarboxylisch ist.

8. Copolymere nach einem der Ansprüche 1 bis 7, **dadurch gekennzeichnet, dass** das nicht-polycarboxylische Nicht-Saccharid-Polymer aus Poly(vinylacetat), Poly(vinylalkohol), den Poly(acrylestern), den Poly(methacrylestern), den Poly(methacrylamiden) und den Poly(acrylamiden) ausgewählt ist.

9. Copolymere nach einem der Ansprüche 1 bis 6, **dadurch gekennzeichnet, dass** das Nicht-Saccharid-Polymer polycarboxylisch ist.

10. Copolymere nach einem der Ansprüche 1 bis 6 oder 9, **dadurch gekennzeichnet, dass** das Nicht-Saccharid-Polymer ein polycarboxylisches Acryl-Polymer ist.

11. Copolymere nach Anspruch 10, **dadurch gekennzeichnet, dass** das polycarboxylische Acryl-Polymer Poly(acrylsäure) oder Poly(methacrylsäure) ist.

12. Copolymere nach einem der Ansprüche 1 bis 11, bei denen das Vernetzungsmittel aus den Diaminen, den natürlichen oder synthetischen Aminosäure oder den Polyaminen und vorzugsweise aus den Diaminen ausgewählt ist.

13. Copolymere nach Anspruch 12, bei denen das Vernetzungsmittel ein Diamin ist.

14. Copolymere nach einem der Ansprüche 1 bis 13, **dadurch gekennzeichnet, dass** das Polysaccharid durch die Mikrobenflora des Kolon abbaubar ist.

15. Copolymere nach Anspruch 14, **dadurch gekennzeichnet, dass** das Polysaccharid aus Chondroitinsulfat, Hyaluronsäure, Pectinsäure, Heparin, Dextran, Chitosan, Amylose, Pectin, den Alginaten oder Xanthan ausgewählt ist.

16. Copolymere nach einem der Ansprüche 14 bis 15, **dadurch gekennzeichnet, dass** das Polysaccharid Chondroitinsulfat ist, das andere Nicht-Saccarid-Polymer Poly(acrylsäure) oder Poly(methacrylsäure) ist und das Vernetzungsmittel Hexandiamin ist.

17. Verfahren zur Herstellung von vernetzten Copolymeren nach einem der Ansprüche 1 bis 16, **dadurch gekennzeichnet, dass** man die nicht vernetzten polycarboxylischen Copolymere in Gegenwart eines Aktivators und des Vernetzungsmittels in wässrigem Medium reagieren läßt.

18. Verfahren nach Anspruch 17, bei dem der Aktivator aus Carbodiimiden, Derivaten der Chinoline und gemischten Anhydriden ausgewählt ist.

19. Verfahren zur Herstellung von nicht vernetzten Copolymeren nach Anspruch 1, **dadurch gekennzeichnet, dass** man auf das Polysaccharid in wässrigem Medium unter Inertatmosphäre und in Gegenwart eines Katalysators das Monomer des Nicht-Saccharid-Polymers aufpfropft, wobei dieses Monomer dann bei diesen Reaktionsbedingungen polymerisiert.

20. Pharmazeutische Zusammensetzung, die mindestens einen Wirkstoff und als inerten Träger oder Hilfsstoff mindestens ein vernetztes Copolymer nach einem der Ansprüche 1 bis 13 enthält.

21. Pharmazeutische Zusammensetzung, die mindestens einen Wirkstoff und als inerten Träger oder Hilfsstoff mindestens tens ein Copolymer nach einem der Ansprüche 14 bis 16 enthält.

22. Verwendung einer pharmazeutischen Zusammensetzung nach einem der Ansprüche 20 bis 21 für eine gesteuerte Freisetzung.

23. Verwendung einer pharmazeutischen Zusammensetzung nach einem der Ansprüche 20 bis 21 als bioadhäsives pharmazeutisches System.

24. Verwendung einer pharmazeutischen Zusammensetzung nach Anspruch 21 für eine spezifische Freisetzung des Wirkstoffs im Kolonbereich.

25. Verwendung nach Anspruch 24 zum Transport des Wirkstoffs, der für die Behandlung der Krankheiten des Kolon bestimmt ist.

26. Verwendung nach Anspruch 24 zum Transport des Wirkstoffs, der im Kolonbereich absorbiert wird.

27. Verwendung nach Anspruch 24 zum Transport des Wirkstoffs, der in den oberen Teilen des Verdauungstrakts abgebaut wird.

## Claims

1. Cross-linked copolymers based on non cross-linked polycarboxylic copolymers and a cross-linking agent comprising at least two amine functions,
each non cross-linked polycarboxylic copolymer comprising at least one non cross-linked polysaccharide linked by a covalent bond to at least one other non saccharidic non cross-linked polymer, and
at least one of the non saccharidic polysaccharides and polymers, constituting the same non cross-linked copolymer, is polycarboxylic.

2. Copolymers according to claim 1, **characterized in that** the polysaccharide is non polycarboxylic.

3. Copolymers according to one of claims 1 to 2, **characterized in that** the non polycarboxylic polysaccharide is chosen from agarose, agaropectin, amylose, amylopectin, arabinogalactan, carrageenans, cellulose or methylcellulose, chitosan, dextran, keratan sulphate, fucans and fucoidans, tragacanth, arabic, locust bean and guar gums or pullulan.

4. Copolymers according to claim 1, **characterized in that** the polysaccharide is polycarboxylic.

5. Copolymers according to one of claims 1 or 4, **characterized in that** the polycarboxylic polysaccharide is chosen from the glycosaminoglycanes, pectinic or alginic acid.

6. Copolymers according to one of claims 1, 4 or 5, **characterized in that** the polycarboxylic polysaccharide is a glycosaminoglycane chosen from hyaluronic acid, chondroitin sulphate, heparin, dermatan sulphate and heparan sulphate.

7. Copolymers according to claim 1 to 6, **characterized in that** the non saccharidic polymer is non polycarboxylic.

8. Copolymers according to one of claims 1 to 7, **characterized in that** the non polycarboxylic non saccharidic polymer is chosen from poly(vinyl acetate), poly(vinyl alcohol), poly(acrylic esters), poly(methacrylic esters), poly(methacrylamides) and poly(acrylamides).

9. Copolymers according to any one of claims 1 to 6, **characterized in that** the non saccharidic polymer is polycarboxylic.

10. Copolymers according to any one of claims 1 to 6 or 9, **characterized in that** the non saccharidic polymer is a polycarboxylic acrylic polymer.

11. Copolymers according to claim 10, **characterized in that** the polycarboxylic acrylic polymer is poly(acrylic acid) or poly(methacrylic acid).

12. Copolymers according to any one of claims 1 to 11, in which the cross-linking agent is chosen from diamines, natural or synthetic amino acids or polyamines, and preferentially diamines.

13. Copolymers according to claim 12 in which the cross-linking agent is a diamine.

14. Copolymers according to one of claims 1 to 13, **characterized in that** the polysaccharide is degradable by the microbial flora of the colon.

15. Copolymers according to claim 14, **characterized in that** the polysaccharide is chosen from chondroitin sulphate, hyaluronic acid, pectinic acid, heparin, dextran, chitosan, amylose, pectin, alginates or xanthan.

16. Copolymers according to any one of claims 14 to 15, **characterized in that** the polysaccharide is chondroitin sulphate, the other said non saccharidic polymer is poly(acrylic acid) or poly(methacrylic acid), and the cross-linking agent is hexanediamine.

17. Process for the preparation of cross-linked copolymers according to any one of claims 1 to 16, **characterized in that** said non cross-linked polycarboxylic copolymers are reacted, in an aqueous medium, in the presence of an activator and of said cross-linking agent.

18. Process according to claim 17, in which the activator is chosen from carbodiimides, quinoline derivatives and mixed anhydrides.

19. Process for the preparation of non cross-linked copolymers according to claim 1, **characterized in that** the monomer of the non saccharidic polymer is grafted onto the polysaccharide in an aqueous medium, under an inert atmosphere and in the presence of a catalyst, which monomer will then polymerise under these reaction conditions.

20. Pharmaceutical composition containing at least one active ingredient and, as an inert support or excipient, at least one cross-linked copolymer according to one of claims 1 to 13.

21. Pharmaceutical composition containing at least one active ingredient and, as an inert support or excipient, at least one copolymer according to one of claims 14 to 16.

22. Use of a pharmaceutical composition according to one of claims 20 to 21 for sustained release.

23. Use of a pharmaceutical composition according to one of claims 20 to 21 as a bioadhesive pharmaceutical system.

24. Use of a pharmaceutical composition according to claim 21 for the specific release of the active ingredient at the level of the colon.

25. Use according to claim 24 to convey the active ingredient intended for the treatment of diseases of the colon.

26. Use according to claim 24 to convey the active ingredient which is absorbed at the level of the colon.

27. Use according to claim 24 to convey the active ingredient which is degraded in the upper parts of the digestive tract.
